(19) Europäisches Patentamt
European Patent Office
Office européen des brevets

(11) **EP 4 741 798 A1**

(12) **EUROPEAN PATENT APPLICATION**

(43) Date of publication:
**13.05.2026 Bulletin 2026/20**

(21) Application number: **25210470.8**

(22) Date of filing: **22.10.2025**

(51) International Patent Classification (IPC):
**G01N 11/02** $^{(2006.01)}$    **G06N 3/088** $^{(2023.01)}$

(52) Cooperative Patent Classification (CPC):
**G01N 11/02; G06N 3/088**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **11.11.2024 KR 20240158919**

(71) Applicant: **SAMSUNG SDI CO., LTD.**
**Yongin-si, Gyeonggi-do 17084 (KR)**

(72) Inventors:
• **KIM, Bora**
**17084 Yongin-si (KR)**

• **KIM, Sungsoo**
**17084 Yongin-si (KR)**
• **LEE, Jaehyoun**
**17084 Yongin-si (KR)**
• **JEON, Keonghee**
**17084 Yongin-si (KR)**
• **JO, Seonggwan**
**17084 Yongin-s (KR)**

(74) Representative: **Maiwald GmbH**
**Elisenhof**
**Elisenstraße 3**
**80335 München (DE)**

(54) **SLURRY PRESSURIZING AND FILTERING APPARATUS FOR ANALYZING SLURRY AND METHOD OF ANALYZING SLURRY**

(57)    A method of analyzing a slurry, which is performed by a processor of a slurry pressurizing and filtering apparatus, may include collecting a cumulative discharge amount of a slurry of an active material for a secondary battery at preset time intervals, calculating a discharge amount per hour of the slurry by using a cumulative discharge amount of the slurry at a previous time and a cumulative discharge amount of the slurry at a current time, classifying state information of the slurry based on the cumulative discharge amount of the slurry and the discharge amount per hour of the slurry, and deriving a discharge rate, which represents flow characteristics and a dispersion state of the slurry, as an evaluation characteristic value based on a result of classifying the state information of the slurry.

FIG. 5

```
                    ( START )
                        │
 COLLECT CUMULATIVE DISCHARGE AMOUNT OF SLURRY ── 510
                        │
 CALCULATE DISCHARGE AMOUNT PER HOUR OF SLURRY ── 520
                        │
    CLASSIFY STATE INFORMATION OF SLURRY ── 530
                        │
 DERIVE EVALUATION CHARACTERISTIC VALUE OF SLURRY ── 540
                        │
                     ( END )
```

EP 4 741 798 A1

**Description**

**BACKGROUND**

**1. Field**

Embodiments of the present disclosure relate to a slurry pressurizing and filtering apparatus to analyze a slurry of an active material for a secondary battery, and a method of analyzing a slurry of an active material for a secondary battery, which is performed by the slurry pressurizing and filtering apparatus.

**2. Description of the Related Art**

In processes of industrially handling slurries of active materials for secondary batteries, the continuous flow characteristics of the slurries are very important. This is because the design of the entire facility, such as the design of the length, thickness, and angle of production line pipes, may be determined based on the continuous flow characteristics of slurries of active materials for secondary batteries.

In existing slurry pressurizing and filtering apparatuses, due to the high viscosity of slurries, there is a possibility that abnormal values may randomly occur during data measurement. Thus, the reliability of discharge amount and discharge rate data provided by a slurry pressurizing and filtering apparatus may be lowered.

The above information disclosed in this background section is only for enhancement of understanding of the background of the present disclosure and therefore it may contain information that does not constitute prior art.

**SUMMARY**

Embodiments of the present disclosure are directed to classifying data measured in a slurry pressurizing and filtering apparatus into a normal value and an abnormal value and removing the abnormal value.

Embodiments of the present disclosure are directed to deriving a standardized evaluation characteristic value of a slurry by using a normal value.

However, the technical objects of embodiments of the present disclosure are not limited to the above, and other objects of embodiments that are not described herein will be clearly understood by those skilled in the art from reviewing the following disclosure.

According to an aspect, a method of analyzing a slurry, which is performed by a processor of a slurry pressurizing and filtering apparatus, includes collecting a cumulative discharge amount of a slurry of an active material for a secondary battery at preset time intervals, calculating a discharge amount per hour of the slurry by using a cumulative discharge amount of the slurry at a previous time and a cumulative discharge amount of the slurry at a current time, classifying state information of the slurry based on the cumulative discharge amount of the slurry and the discharge amount per hour of the slurry, and deriving a discharge rate, which represents flow characteristics and a dispersion state of the slurry, as an evaluation characteristic value based on a result of classifying the state information of the slurry.

According to an aspect, a slurry pressurizing and filtering apparatus to analyze a slurry includes one or more processors, and a memory operatively connected to the one or more processors and configured to store at least one code executed by the processor, wherein the memory is configured to store the at least one code to, if (e.g., when) executed by the processor, cause the processor to collect a cumulative discharge amount of a slurry of an active material for a secondary battery at preset time intervals, calculate a discharge amount per hour of the slurry by using a cumulative discharge amount of the slurry at a previous time and a cumulative discharge amount of the slurry at a current time, classify state information of the slurry based on the cumulative discharge amount of the slurry and the discharge amount per hour of the slurry, and derive a discharge rate, which represents flow characteristics and a dispersion state of the slurry, as an evaluation characteristic value based on a result of classifying the state information of the slurry.

In embodiments, other methods to implement the subject matter of the present disclosure, other systems, and a computer-readable recording medium on which a computer program to execute the method is stored may be further provided.

Other aspects and features of embodiments other than those described above will become apparent from the following drawings, claims, and detailed description of the disclosure.

**BRIEF DESCRIPTION OF THE DRAWINGS**

The accompanying drawings illustrate embodiments of the present disclosure and, together with the following detailed description, serve to provide further understanding of the technical scope of the present disclosure. However, the present disclosure is not to be construed as being limited to the details shown in the drawings, in which:

FIG. 1 illustrates a configuration of a slurry pressurizing and filtering apparatus to analyze a slurry according to an embodiment of the present disclosure;

FIG. 2 illustrates a configuration of a processor in the slurry pressurizing and filtering apparatus to analyze a slurry according to an embodiment of the present disclosure;

FIG. 3 shows example diagrams illustrating a classification unit in the processor according to an embodiment of the present disclosure;

FIG. 4 shows example diagrams illustrating a derivation unit in the processor according to an embodiment of the present disclosure; and

FIG. 5 is a flowchart illustrating a method of analyzing a slurry, which is performed by a processor of a slurry pressurizing and filtering apparatus according to an embodiment of the present disclosure.

## DETAILED DESCRIPTION

Hereinafter, embodiments of the present disclosure will be described with reference to the accompanying drawings. Prior to the following description, it should be understood that the terms used in the specification and the appended claims should not be construed as limited to general and dictionary meanings, but interpreted based on the meanings and concepts corresponding to technical aspects of the present disclosure on the basis of the principle that the inventor is allowed to act as their own lexicographer and define terms suitably or appropriately for the best description. Accordingly, embodiments disclosed in the present specification and configurations illustrated in the drawings are merely example embodiments of the present disclosure and do not represent all of the technical ideas of the present disclosure, and thus it should be understood that there may be various suitable equivalents and modifications that may substitute these at the time of filing of the present application. Further, "comprise and include" and/or "comprising and including" used in this specification should be interpreted as specifying the presence of described shapes, numbers, steps, operations, members, elements, and/or groups thereof and do not exclude the presence or addition of other shapes, numbers, operations, members, elements, and/or groups thereof. Further, the use of "may" and "may be" if (e.g., when) describing embodiments of the present disclosure refers to "one or more embodiments of the present disclosure."

In embodiments, for a better understanding of the present disclosure, the accompanying drawings may not be illustrated on an actual scale and sizes of some elements may be exaggerated. In embodiments, the same reference numbers may be assigned to the same components in different embodiments.

Stating that two objects of comparison are "the same" means that the two objects of comparison are "substantially the same." Therefore, substantially the same may include a deviation that is considered low in the art, for example, a deviation of 5% or less. In embodiments, uniformity of a parameter in a certain area may mean uniformity from an average perspective.

It will be understood that, although the terms first, second, and the like are used herein to describe various components, these components should not be limited by these terms. These terms are only used to distinguish one component from another component, and a first component may also be a second component unless particularly described otherwise.

Through the specification, each component may be singular or plural unless particularly described otherwise.

Arrangement of any component on the "top (or bottom)" of a component or on the "top (or bottom)" of a component includes not only the arrangement in which any component is disposed in contact with the top (or bottom) of the component, but also the arrangement in which that other components may be interposed between the component and any component disposed on (or under) the component.

Also, if (e.g., when) it is said that a certain element is "connected" or "coupled" to another component, this may mean that the components are directly connected or coupled to each other, but it should be understood that another component may be "interposed" between the components or the components may be "connected" or "coupled" to each other via another component. Further, the term "electrically coupled" may mean not only "directly coupled" but also may include "coupled via other interposing component."

If (e.g., when) reference is made throughout the specification to "A and/or B," this means A, B, or A and B, unless otherwise specified. For example, "and/or" includes all or any combination of a plurality of listed items. "C to D" refers to C or more and D or less unless particularly described otherwise.

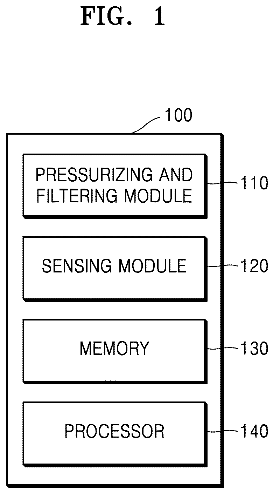

FIG. 1 illustrates a configuration of a slurry pressurizing and filtering apparatus 100 to analyze a slurry according to an embodiment of the present disclosure. Referring to FIG. 1, the slurry pressurizing and filtering apparatus 100 for analyzing the slurry (hereinafter referred to as a slurry pressurizing and filtering apparatus) may include a pressurizing and filtering module 110, a sensing module 120, a memory 130, and a processor 140.

The pressurizing and filtering module 110 may apply a set or specific pressure to a slurry to allow the slurry to pass through a filter. In the present embodiment, the pressurizing and filtering module 110 may use a pump to apply a set or specific pressure to the slurry and send the slurry to the filter. Filters may usually include a fine mesh and/or porous medium and may allow only suitable or desired particles having a suitable or desired size to pass therethrough and may block larger particles. The slurry may pass through the filter so that impurities may be removed.

The sensing module 120 may be provided at an output portion of the pressurizing and filtering module 110 and may sense a cumulative discharge amount of a slurry passing through the filter. The sensing module 120 may calculate the cumulative discharge amount of the slurry at preset time intervals (for example, 1 second). In the present embodiment, the sensing module 120 may include at least one selected from a flow meter and a load cell. The flow meter may detect a flow rate and amount of a slurry passing through the filter. The load cell may detect a cumulative discharge amount by measuring a weight of a slurry passing through the filter.

The memory 130 may store data used for slurry analysis. In the present embodiment, the memory 130 may store a cumulative discharge amount detected by the sensing module 120. In some embodiments, the memory 130 may store a result of calculating a discharge amount per hour processed by the processor 140, a result of generating a first graph, a result of classifying state information of a slurry, a result of calculating a discharge rate per hour, and a result of deriving an evaluation characteristic value. In some embodiments, an artificial intelligence algorithm to classify state information of a slurry may be stored in the memory 130.

In the present embodiment, the memory 130 may be operably connected to the processor 140 and may store at least one code associated with an operation performed by the processor 140.

In some embodiments, the memory 130 may perform a function of temporarily or permanently storing data processed by the processor 140. In embodiments, the memory 130 may include a magnetic storage medium and/or a flash storage medium, but the scope of the present disclosure is not limited thereto. The memory 130 may include an internal memory and/or an external memory and may include a volatile memory such as a dynamic random access memory (DRAM), a static RAM (SRAM), and/or a synchronous DRAM (SDRAM) and a non-volatile memory such as a one time programmable read-only memory (OTPROM), a programmable read-only memory (PROM), an erasable PROM (EPROM), an electrically erasable PROM (EEPROM), a mask ROM, a flash ROM, a NAND flash memory, and/or a NOR flash memory, a flash drive such as a solid state drive (SSD), a compact flash (CF) card, a secure digital (SD) card, a micro-SD card, a mini-SD card, an extreme digital (xD) card, and/or a memory stick, and/or a storage device such as a hard disk drive (HDD).

The processor 140 may collect a cumulative discharge amount of a slurry at preset time intervals from the sensing module 120 and may calculate a discharge amount per hour of the slurry. The processor 140 may classify state information of the slurry based on the cumulative discharge amount of the slurry and the discharge amount per hour of the slurry. The processor 140 may derive a discharge rate, which represents the flow characteristics and dispersion state of the slurry, as an evaluation characteristic value based on a result of classifying the state information of the slurry.

In the present embodiment, the processor 140 may process instructions of a computer program by performing basic arithmetic, logic, and input/output operations. In some embodiments, the processor 140 may control the overall operations of other components associated with the pressurizing and filtering module 110.

For example, the processor 140 may perform at least some of data analyzing, processing, and result information generating to perform the above-described operations by using at least one selected from a machine learning, a neural network, and a deep learning algorithm as a rule-based or artificial intelligence algorithm. Examples of a neural network may include models such as a convolutional neural network (CNN), a deep neural network (DNN), and a recurrent neural network (RNN).

For example, the processor 140 may be implemented as an array of a plurality of logic gates or may also be implemented as a combination of a general-purpose microprocessor and a memory storing a program that may be executed on a microprocessor. For example, the processor 140 may include a general-purpose processor, a central processing unit (CPU), a microprocessor, a digital signal processor (DSP), a controller, a microcontroller, a state machine, and/or the like. In some embodiments, the processor 140 may include an application-specific semiconductor (ASIC), a programmable logic device (PLD), a field programmable gate array (FPGA), and/or the like. For example, the processor 140 may refer to a combination of processing devices such as a combination of a DSP and a microprocessor, a combination of a plurality of microprocessors, a combination of one or more microprocessors in conjunction with a DSP core, or a combination of any suitable other such configurations.

In the present embodiment, the pressurizing and filtering apparatus 100 may further include a communication unit. The communication unit may transmit data processed by the processor 140 to an external device (for example, a user terminal) in conjunction with a network. In an embodiment, under the control of the processor 140, the communication unit may transmit data processed in a process of deriving a cumulative discharge amount of a slurry, a discharge amount per hour of the slurry, and a result of classifying state information of the slurry, and a result of deriving an evaluation characteristic value of the slurry to the user terminal.

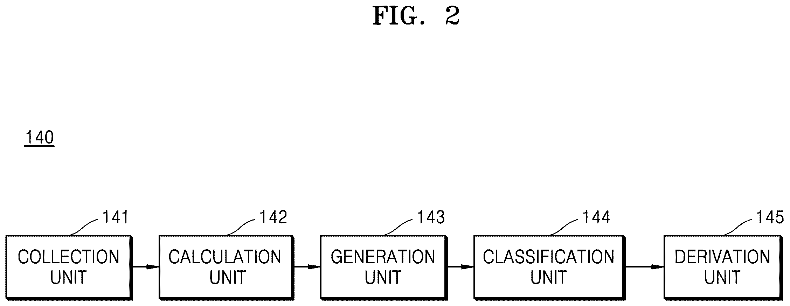

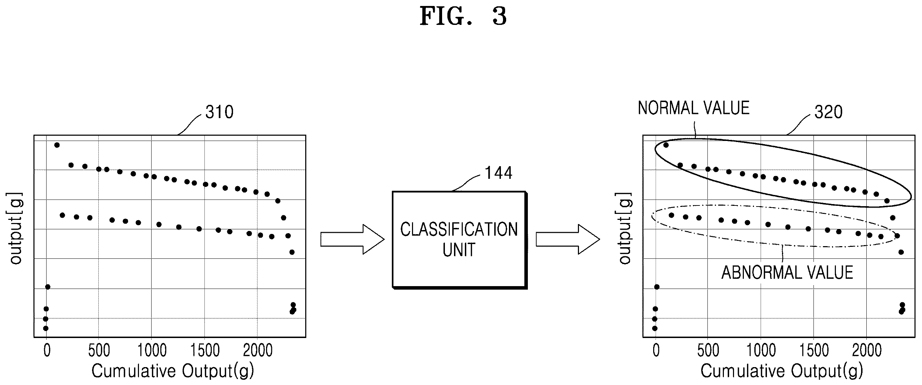

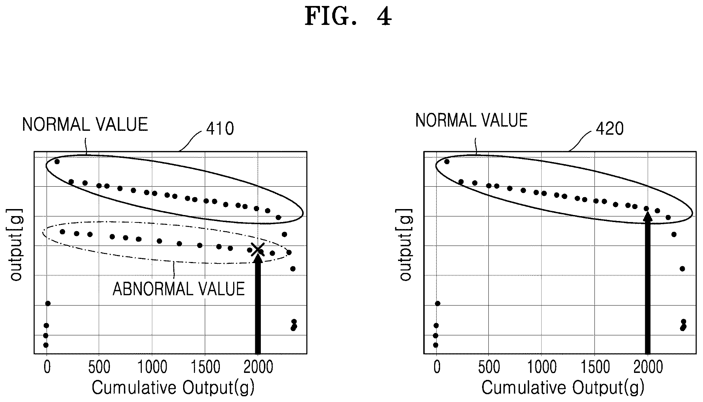

FIG. 2 illustrates a configuration of the processor 140 in the slurry pressurizing and filtering apparatus 100 to analyze a slurry according to an embodiment of the present disclosure. FIG. 3 shows example diagrams illustrating a classification unit 144 in the processor 140 according to an embodiment of the present disclosure. FIG. 4 shows example diagrams illustrating a derivation unit 145 in the processor 140 according to an embodiment of the present disclosure. In the following description, parts that overlap those described with reference to FIG. 1 may not be repeated.

Referring to FIGS. 2 to 4, the processor 140 may include a collection unit 141, a calculation unit 142, a generation unit 143, the classification unit 144, and the derivation unit 145.

The collection unit 141 may collect a cumulative discharge amount (cumulative output g) of a slurry at preset time intervals (for example, 1 second). After a preset pressure is applied to a slurry, the collection unit 141 may collect a cumulative discharge amount of a slurry passing through the filter from the sensing module 120 (at least one selected from the flow meter and the load cell).

The calculation unit 142 may calculate a discharge amount (cumulative output g) per hour (for example, 1 second) of the slurry based on the cumulative discharge amount of the slurry. The calculation unit 142 may calculate a difference value between a cumulative discharge amount of a slurry at a previous time t-1 and a cumulative discharge amount of a slurry at a current time t as the discharge amount per hour.

The generation unit 143 may generate a first graph by plotting the cumulative discharge amount (cumulative output g) of the slurry on an X-axis and the discharge amount (cumulative output g) per hour of the slurry corresponding to the X-axis on a Y-axis. A discharge amount per hour corresponding to any one cumulative discharge amount may be shown on the first graph. The discharge characteristics of a slurry over time may be analyzed through the first graph.

The classification unit 144 may classify state information of the slurry based on the cumulative discharge amount of the slurry and the discharge amount per hour of the slurry. The classification unit 144 may classify the state information of the slurry corresponding to the cumulative discharge amount of the slurry and the discharge amount per hour of the slurry by using an unsupervised learning-based deep learning model that is trained in advance to classify the state information of the slurry by inputting the cumulative discharge amount of the slurry and the discharge amount per hour of the slurry. In the present embodiment, the state information of the slurry may include a normal value and an abnormal value.

In the present embodiment, the unsupervised learning-based deep learning model may use one or more algorithms such as an auto encoder, K-means clustering, a self-organizing map, a deep belief network, and/or a support vector machine. In some embodiments, according to the types or kinds of algorithms, state information of the slurry that is finally output from the unsupervised learning-based deep learning model may be classified into a normal value and an abnormal value and may also be expressed in the form of a mean squared error (MSE) that indicates how much the state information deviates from a normal value.

FIG. 3 shows a first graph 310 including a cumulative discharge amount of a slurry and a discharge amount per hour of the slurry. The classification unit 144 may generate a result 320, in which a normal value and an abnormal value are classified, by using the first graph 310 as an input. The result 320 in which the normal value and the abnormal value are classified may be an example of an output result of a K-means clustering model and may be an example in which data is classified into one of two groups and displayed (k=2). The K-means clustering model may perform a task of classifying the cumulative discharge amount of the slurry and the discharge amount per hour of the slurry into the nearest group of two groups.

The derivation unit 145 may derive a discharge rate, which represents the flow characteristics and dispersion state of the slurry, as an evaluation characteristic value based on a result of classifying the state information of the slurry. In the present embodiment, the derivation unit 145 may remove an abnormal value from the result of classifying the state information of the slurry received from the classification unit 144 and may use a normal value.

The derivation unit 145 may calculate a discharge rate (output rate [%]) per hour of the slurry based on the discharge amount per hour of the slurry included in the normal value and a first reference value. In the present embodiment, the first reference value may represent a discharge amount per hour corresponding to a cumulative discharge amount (for example, in a range of 310 g to 500 g in FIG. 3) selected from the cumulative discharge amount of the slurry.

An equation to calculate a discharge rate per hour of a slurry may be Equation 1 below.

Equation 1

Output rate [%] = discharge amount (output [g]) per hour/first reference value × 100

The derivation unit 145 may generate a second graph by plotting the cumulative discharge amount (cumulative output g) of the slurry on an X-axis and the discharge amount (output rate [%]) per hour of the slurry corresponding to the X-axis on a Y-axis. A discharge rate per hour corresponding to any one cumulative discharge amount may be shown on the second graph. The discharge rate characteristics of a slurry over time may be analyzed through the second graph.

The derivation unit 145 may determine the discharge rate per hour of the slurry, which corresponds to a second reference value (for example, 2,000 g) representing a maximum value of the cumulative discharge amount on the second graph, as an evaluation characteristic value.

FIG. 4 shows a 2-1 graph 410 including a cumulative discharge amount of a slurry and a discharge rate per hour of the slurry in which abnormal values and normal values are included, and a 2-2 graph 420 including a cumulative discharge amount of a slurry and a discharge rate per hour of the slurry in which abnormal values are removed, and only normal values are included. The second graph described above in the present embodiment may be specified as the 2-2 graph 420.

If (e.g., when) an evaluation characteristic value is determined by using the 2-1 graph 410, the reliability of the evaluation characteristic value may be reduced because the evaluation characteristic value is selected as an abnormal value. In the present embodiment, however, because the evaluation characteristic value may be determined by using the 2-2 graph 420, and the evaluation characteristic value is selected as a normal value, the reliability of the evaluation characteristic value may be improved.

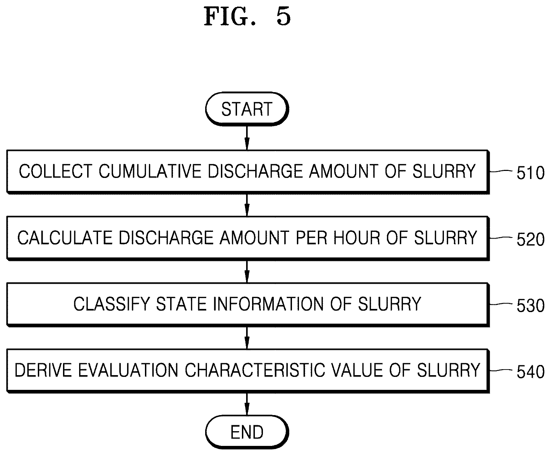

FIG. 5 is a flowchart illustrating a method of analyzing a slurry, which is performed by a processor of a slurry pressurizing and filtering apparatus according to an embodiment of the present disclosure. In the following description, parts that overlap those described with reference to FIGS. 1 and 4 may not be repeated. The following will be described on the assumption that the method of analyzing a slurry according to the present embodiment is performed by the processor 140 of the pressurizing and filtering apparatus 100 with the help of peripheral components.

In operation S510, the processor 140 may collect a cumulative discharge amount of a slurry at preset time intervals. In the present embodiment, the processor 140 may collect a cumulative discharge amount of a slurry, which passes through the filter after a preset pressure is applied, from at least one selected from the flow meter and the load cell.

In operation S520, the processor 140 may calculate a discharge amount per hour of the slurry by using a cumulative discharge amount of the slurry at a previous time $t-1$ and a cumulative discharge amount of the slurry at a current time $t$.

In the present embodiment, the processor 140 may generate a first graph by plotting the cumulative discharge amount (cumulative output g) of the slurry on an X-axis and the discharge amount (cumulative output g) per hour of the slurry corresponding to the X-axis on a Y-axis.

In operation S530, the processor 140 may classify state information of the slurry based on the cumulative discharge amount of the slurry and the discharge amount per hour of the slurry which are included in the first graph. In the present embodiment, the processor 140 may classify state information of the slurry corresponding to the cumulative discharge amount of the slurry and the discharge amount per hour of the slurry by using an unsupervised learning-based deep learning model that is trained in advance to classify the state information of the slurry by inputting the cumulative discharge amount of the slurry and the discharge amount per hour of the slurry. In embodiments, the state information of the slurry may be classified into a normal value and an abnormal value, and the processor 140 may remove the abnormal value from the state information of the slurry including the normal value and the abnormal value.

In operation S540, the processor 140 may derive a discharge rate, which represents the flow characteristics and dispersion state of the slurry, as an evaluation characteristic value based on a result of classifying the state information of the slurry. In the present embodiment, the processor 140 may remove the abnormal value from the result of classifying the state information of the slurry and may apply the normal value. The processor 140 may calculate a discharge rate per hour of the slurry by using a discharge amount per hour of the slurry based on the normal value and a first reference value representing a discharge amount per hour from a cumulative discharge amount selected from the cumulative discharge amount of the slurry. The processor 140 may generate a second graph by plotting the cumulative discharge amount (cumulative output g) of the slurry on an X-axis and a discharge amount (output rate %) per hour of the slurry corresponding to the X-axis on a Y-axis. The processor 140 may determine a discharge rate per hour of the slurry, which corresponds to a second reference value representing a maximum value of the cumulative discharge amount on the second graph, as an evaluation characteristic value.

According to embodiments of the present disclosure, by removing an abnormal value from data measured in a slurry pressurizing and filtering apparatus, the reliability of measured data may be improved.

In some embodiments, by removing an abnormal value from data measured in a slurry pressurizing and filtering apparatus, errors in analysis results may be minimized or reduced, thereby providing more accurate information for process determination or adjustment.

In some embodiments, by standardizing the characteristic values of a slurry, the slurry of each batch may be maintained at the same quality, ensuring or increasing consistency in a process.

In some embodiments, by removing an abnormal value from data measured in a slurry pressurizing and filtering apparatus and standardizing characteristic values, data processing deviations for each operator that may occur in an existing method may be eliminated, and a data processing time may be reduced.

However, the effects that may be achieved through embodiments of the present disclosure are not limited to the above-described effects, and other technical effects that are not described herein will be clearly understood by those skilled in the art from reviewing the present disclosure.

**Claims**

1. A method of analyzing a slurry, which is performed by a processor (140) of a slurry pressurizing and filtering apparatus, the method comprising:

collecting (510) a cumulative discharge amount of a slurry of an active material for a secondary battery at preset time intervals;

calculating (520) a discharge amount per hour of the slurry by using a cumulative discharge amount of the slurry at a previous time and a cumulative discharge amount of the slurry at a current time;

classifying (530) state information of the slurry based on the cumulative discharge amount of the slurry and the discharge amount per hour of the slurry; and

deriving (540) a discharge rate, which represents flow characteristics and a dispersion state of the slurry, as an evaluation characteristic value based on a result (320) of classifying the state information of the slurry.

2. The method as claimed in claim 1, wherein the collecting of the cumulative discharge amount of the slurry comprises collecting the cumulative discharge amount of the slurry, which passes through a filter after a preset pressure is applied, from at least one selected from a flow meter and a load cell.

3. The method as claimed in claim 1 or 2, further comprising, after the collecting of the discharge amount per hour of the slurry, generating a first graph (310) by plotting the cumulative discharge amount of the slurry on an X-axis and plotting the discharge amount per hour of the slurry corresponding to the X-axis on a Y-axis.

4. The method as claimed in any one of the preceding claims, wherein the classifying of the state information of the slurry comprises classifying the state information of the slurry corresponding to the cumulative discharge amount of the slurry and the discharge amount per hour of the slurry by using an unsupervised learning-based deep learning model that is trained in advance to classify the state information of the slurry by inputting the cumulative discharge amount of the slurry and the discharge amount per hour of the slurry.

5. The method as claimed in claim 4, wherein the state information of the slurry is classified into a normal value and an abnormal value, and

the method further comprises, after the classifying of the state information of the slurry, removing the abnormal value from the state information of the slurry.

6. The method as claimed in any one of the preceding claims, wherein the deriving of the evaluation characteristic value comprises:

calculating a discharge rate per hour of the slurry by using the discharge amount per hour of the slurry and a first reference value representing a discharge amount per hour in a cumulative discharge amount selected from the cumulative discharge amount of the slurry;

generating a second graph by plotting the cumulative discharge amount of the slurry on an X-axis and plotting the discharge rate per hour of the slurry corresponding to the X-axis on a Y-axis; and

determining the discharge rate per hour of the slurry, which corresponds to a second reference value representing a maximum value of the cumulative discharge amount on the second graph, as the evaluation characteristic value.

7. The method as claimed in claim 6, further comprising, before the calculating of the discharge rate per hour of the slurry, removing an abnormal value from the result (320) of classifying the state information of the slurry, and applying a normal value.

8. A computer-readable recording medium having recorded thereon a program that causes the method as claimed in claim 1 to be executed on a computer.

9. A slurry pressurizing and filtering apparatus comprising:

one or more processors (140); and

a memory (130) operatively connected to the one or more processors (140) and configured to store at least one code executed by the one or more processors (140),

wherein the memory (130) is configured to store the at least one code to, if executed by the one or more processors (140), cause the one or more processors (140) to:

collect a cumulative discharge amount of a slurry of an active material for a secondary battery at preset time intervals;

calculate a discharge amount per hour of the slurry by using a cumulative discharge amount of the slurry at a previous time and a cumulative discharge amount of the slurry at a current time;
classify state information of the slurry based on the cumulative discharge amount of the slurry and the discharge amount per hour of the slurry; and
derive a discharge rate, which represents flow characteristics and a dispersion state of the slurry, as an evaluation characteristic value based on a result (320) of classifying the state information of the slurry.

10. The slurry pressurizing and filtering apparatus as claimed in claim 9, wherein the memory (130) is configured to store the at least one code to cause the one or more processors (140) to, if collecting the cumulative discharge amount of the slurry, collect the cumulative discharge amount of the slurry, which passes through a filter after a preset pressure is applied, from at least one selected from a flow meter and a load cell.

11. The slurry pressurizing and filtering apparatus as claimed in claim 9 or 10, wherein the memory (130) is configured to store the at least one code to cause the one or more processors (140) to, after collecting the discharge amount per hour of the slurry, generate a first graph (310) by plotting the cumulative discharge amount of the slurry on an X-axis and plotting the discharge amount per hour of the slurry corresponding to the X-axis on a Y-axis.

12. The slurry pressurizing and filtering apparatus as claimed in any one of the claims 9 to 11, wherein the memory (130) is configured to store the at least one code to cause the one or more processors (140) to, if classifying the state information of the slurry, classify the state information of the slurry corresponding to the cumulative discharge amount of the slurry and the discharge amount per hour of the slurry by using an unsupervised learning-based deep learning model that is trained in advance to classify the state information of the slurry by inputting the cumulative discharge amount of the slurry and the discharge amount per hour of the slurry.

13. The slurry pressurizing and filtering apparatus as claimed in claim 12, wherein the state information of the slurry is classified into a normal value and an abnormal value, and
the memory (130) is configured to store the at least one code to cause the one or more processors (140) to, after classifying the state information of the slurry, remove the abnormal value from the state information of the slurry.

14. The slurry pressurizing and filtering apparatus as claimed in any one of the claims 9 to 13, wherein the memory (130) is configured to store the at least one code to cause the one or more processors (140) to:

when deriving the evaluation characteristic value, calculate a discharge rate per hour of the slurry by using the discharge amount per hour of the slurry and a first reference value representing a discharge amount per hour in a cumulative discharge amount selected from the cumulative discharge amount of the slurry;
generate a second graph by plotting the cumulative discharge amount of the slurry on an X-axis and plotting the discharge rate per hour of the slurry corresponding to the X-axis on a Y-axis; and
determine the discharge rate per hour of the slurry, which corresponds to a second reference value representing a maximum value of the cumulative discharge amount on the second graph, as the evaluation characteristic value.

15. The slurry pressurizing and filtering apparatus as claimed in claim 14, wherein the memory (130) is configured to store the at least one code to cause the one or more processors (140) to, before calculating the discharge rate per hour of the slurry, remove an abnormal value from the result (320) of classifying the state information of the slurry, and apply a normal value.

# FIG. 1

100

| PRESSURIZING AND FILTERING MODULE | 110 |
| SENSING MODULE | 120 |
| MEMORY | 130 |
| PROCESSOR | 140 |

# FIG. 2

<u>140</u>

# FIG. 3

EP 4 741 798 A1

**FIG. 4**

# FIG. 5

```
                    ( START )
                        │
                        ▼
┌──────────────────────────────────────────────────┐
│  COLLECT CUMULATIVE DISCHARGE AMOUNT OF SLURRY     │── 510
└──────────────────────────────────────────────────┘
                        │
                        ▼
┌──────────────────────────────────────────────────┐
│  CALCULATE DISCHARGE AMOUNT PER HOUR OF SLURRY     │── 520
└──────────────────────────────────────────────────┘
                        │
                        ▼
┌──────────────────────────────────────────────────┐
│       CLASSIFY STATE INFORMATION OF SLURRY         │── 530
└──────────────────────────────────────────────────┘
                        │
                        ▼
┌──────────────────────────────────────────────────┐
│  DERIVE EVALUATION CHARACTERISTIC VALUE OF SLURRY  │── 540
└──────────────────────────────────────────────────┘
                        │
                        ▼
                    (  END  )
```

Europäisches
Patentamt

European
Patent Office

Office européen
des brevets

# EUROPEAN SEARCH REPORT

**Application Number**

EP 25 21 0470

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 220 626 101 U (RUIPU LANJUN ENERGY CO LTD) 19 March 2024 (2024-03-19) | 1,3-5,8, 9,11-13 | INV. G01N11/02 |
| Y | * paragraph [0002] * | 2,10 | |
| A | * paragraph [0024] *<br>* paragraph [0034] *<br>* paragraph [0050] - paragraph [0052] *<br>* figures 1-3 * | 6,7,14, 15 | ADD. G06N3/088 |
| Y | CN 206 444 774 U (PHST CORP) 29 August 2017 (2017-08-29) | 2,10 | |
| A | * the whole document *<br>* figures 1,2 * | 1,8,9 | |
| Y | CN 215 574 461 U (SINOMA LITHIUM BATTERY SEPARATOR CO LTD) 18 January 2022 (2022-01-18) | 2,10 | |
| A | * paragraph [0018] - paragraph [0029] *<br>* figures 1-4 * | 1,8,9 | |
| A | CN 208 350 560 U (DONGGUAN TAFEL NEW ENERGY TECH CO LTD ET AL.) 8 January 2019 (2019-01-08)<br>* the whole document *<br>* figures 1-3 * | 1-15 | |

-----

-----

-----

-----

**TECHNICAL FIELDS SEARCHED (IPC)**

G01N
B05B
G06E
G06N

The present search report has been drawn up for all claims

| Place of search | Date of completion of the search | Examiner |
|---|---|---|
| The Hague | 26 February 2026 | Verdoodt, Erik |

EPO FORM 1503 03.82 (P04C01)

## ANNEX TO THE EUROPEAN SEARCH REPORT
## ON EUROPEAN PATENT APPLICATION NO.

EP 25 21 0470

This annex lists the patent family members relating to the patent documents cited in the above-mentioned European search report.
The members are as contained in the European Patent Office EDP file on
The European Patent Office is in no way liable for these particulars which are merely given for the purpose of information.

26-02-2026

| Patent document cited in search report | | Publication date | Patent family member(s) | Publication date |
|---|---|---|---|---|
| CN 220626101 | U | 19-03-2024 | NONE | |
| CN 206444774 | U | 29-08-2017 | NONE | |
| CN 215574461 | U | 18-01-2022 | NONE | |
| CN 208350560 | U | 08-01-2019 | NONE | |

EPO FORM P0459

For more details about this annex : see Official Journal of the European Patent Office, No. 12/82